# FASCICULE DE BREVET EUROPEEN

(11) **EP 0 953 348 B1**
(45) Date de publication et mention de la délivrance du brevet: **04.02.2004**
(21) Numéro de dépôt: 99400673.2
(22) Date de dépôt: 19.03.1999
(51) Int. Cl.: A61K 9/70, A61K 7/00

(54) **Patch à matrice adhesive**
Pflaster mit einer Haftmatrix
Patch with adhesive matrix

(30) Priorité: 24.03.1998 FR 9803589
(43) Date de publication de la demande: 03.11.1999
(73) Titulaire: L'OREAL, 75008 Paris (FR)
(72) Inventeur: Gueret, Jean-Louis, 75016 Paris (FR)
(74) Mandataire: Leszczynski, André

(56) Documents cités:
- EP-A- 0 309 309
- EP-A- 0 353 972
- EP-A- 0 412 869
- EP-A- 0 413 034
- EP-A- 0 651 984

## Description

La présente invention concerne un patch destiné à être appliqué temporairement sur la peau pour exercer sur celle-ci une action cosmétique.

On connaît des patchs comportant une feuille de support revêtue sur une face d'une couche formant réservoir appelée matrice, contenant une ou plusieurs substances actives destinées à diffuser dans la peau et/ou à agir sur celle-ci.

La matrice peut être réalisée dans un matériau présentant des propriétés adhésives intrinsèques ou non, auquel cas le patch comporte un revêtement adhésif à sa surface.

Il faut alors veiller à ce que ce revêtement ne gêne pas l'action de la ou des substances actives contenues dans la matrice.

L'invention concerne plus particulièrement un patch dans lequel la matrice est réalisée dans un matériau présentant des propriétés adhésives intrinsèques.

Le choix de ce matériau soulève des difficultés, à savoir :
- il doit pouvoir contenir la ou les substances actives destinées à agir sur la peau,
- son adhérence sur la peau ne doit pas être trop forte, notamment s'il est d'application répétée, sous peine d'irriter la région du corps sur laquelle le patch est appliqué et de rendre l'enlèvement de ce dernier douloureux,
- son adhérence ne doit pas non plus être trop faible, sous peine de ne pas pouvoir adhérer à la peau si celle-ci est humide ou le devient, par exemple à cause de la transpiration,
- il doit être suffisamment flexible pour permettre au patch d'épouser le relief de la région du corps sur laquelle il est appliqué,
- il doit rester sur la feuille de support lors du retrait du patch,
- enfin, il doit pouvoir permettre l'extraction des impuretés qui se trouvent à la surface de la peau, notamment le sébum ou la transpiration.

Malgré ces difficultés, quelques matériaux ont pu être proposés pour réaliser la matrice, mais ceux-ci ne conviennent pas forcément à toutes les substances actives que l'on souhaite pouvoir y incorporer.

Les brevets français 2 738 744 ou européen 0 309 309 enseignent notamment d'utiliser pour constituer la matrice des matériaux hydrophobes ou hydrosolubles.

Le matériau hydrosoluble décrit dans le brevet européen 0 309 309 est relativement peu pratique d'emploi car il ne présente pas les propriétés adhésives requises avant l'application sur la peau et nécessite l'humidification préalable de celle-ci.

La demande de brevet européen EP-A-0 412 869 décrit un film composite comportant une matrice de polymère silicone accolée à une couche occlusive. La matrice comporte du côté opposé à la couche occlusive une trame constituant une armature et une pellicule de protection amovible. La trame réduit la surface d'adhérence de la pellicule de protection sur la matrice.

Dans un tel film composite, la trame empêche localement la matrice de venir au contact de la peau. Lors du retrait du film, la trame risque de laisser sur la peau une marque d'autant plus prononcée que le matériau constituant la matrice est fortement adhésif. Dans le cas d'une matrice de polymère de silicone, dont le pouvoir adhésif (TAC) est relativement faible (de l'ordre de 100 g/cm²), cet inconvénient n'est toutefois pas trop gênant. Enfin, la trame empêche d'exercer une action mécanique de désincrustation sur toute la surface de la peau recouverte par le film.

La présente invention vise à faciliter le choix du matériau constituant la matrice, sans pour autant compliquer l'utilisation du patch.

L'invention y parvient grâce à un nouveau patch comportant une matrice réalisée dans un matériau ayant des propriétés auto-adhésives avant l'application sur la peau, caractérisé par le fait qu'il comporte une structure perméable entièrement noyée dans ladite matrice à proximité de sa surface, de manière à modifier son pouvoir adhésif global.

Cette structure diffère ainsi de l'art antérieur et notamment du brevet EP-A-0 651 984 qui décrit une structure mufticouche comportant une couche d'adhésif enrobant une feuille poreuse non-tissée, ladite feuille poreuse se situant du côté opposé à la surface de la couche d'adhésif destinée à venir en contact avec la peau.

De préférence, la structure, perméable selon la présente invention est hydrophobe.

De préférence encore, ladite structure perméable est constituée par un film perforé, un tissé ou un non tissé à fibres pleines.

Dans le cas où l'on utilise un tissé ou non tissé à fibres creuses, la porosité des fibres est alors choisie de préférence de manière à ce qu'elles soient sensiblement remplies entièrement par la matrice.

Ainsi, la structure perméable n'est pas apte à absorber l'humidité présente sur la peau ou les actifs éventuellement présents dans la matrice. Ces derniers ne risquent donc pas de s'accumuler dans la structure perméable, ce qui pourrait retarder leur diffusion dans la peau.

De préférence, la structure perméable utilisée est choisie de manière à présenter une possibilité d'élongation réduite dans le sens de la longueur du patch, de manière à faciliter le décollement de ce dernier.

Grâce à l'invention on peut contrôler les propriétés d'adhérence du patch sur la peau, en jouant sur la nature de la structure perméable utilisée et l'épaisseur de matrice située entre la surface destinée à être appliquée sur la peau et la structure perméable.

Cette façon d'agir sur les propriétés adhésives du patch permet d'utiliser pour réaliser la matrice un matériau qui, en l'absence de ladite structure perméable, adhérerait trop fortement à la peau pour convenir à la réalisation du patch recherché.

Ainsi, grâce à l'invention, tout nouveau matériau présentant des propriétés adhésives intrinsèques et intéressant en raison de sa compatibilité physique et chimique avec la ou les substances actives que l'on désire y incorporer peut être utilisé sans avoir à craindre une trop forte adhérence sur la peau.

De plus, la structure perméable utilisée permet la diffusion et/ou l'action de la ou des substances actives contenues dans la matrice vers et/ou sur la surface de la peau.

Le fait que la structure perméable utilisée pour modifier le pouvoir adhésif de la matrice soit entièrement noyée dans le matériau constituant cette dernière permet d'exercer une action mécanique de désincrustation sur toute la surface de peau couverte par le patch si on le souhaite, car toute la surface du patch destinée à venir au contact de la peau est adhésive. Le risque de laisser sur la peau une marque ayant un motif correspondant à celui de la structure perméable utilisée est également réduit. Enfin, lorsque des substances actives sont contenues dans la matrice, celles-ci peuvent exercer leur action sur toute la surface de la peau qui est au contact du patch, alors que dans la demande de brevet européen EP-A-0 412 869 la trame appliquée directement sur la peau gêne dans une certaine mesure la diffusion des actifs dans celle-ci.

D'autres caractéristiques et avantages de la présente invention ressortiront à la lecture de la description détaillée qui va suivre, d'exemples de réalisation de l'invention, et à l'examen du dessin annexé sur lequel :
- la figure 1 est une vue schématique en coupe d'un patch conforme à un premier exemple de réalisation de l'invention, et
- la figure 2 est une vue schématique en coupe d'un patch conforme à un deuxième exemple de réalisation de l'invention.

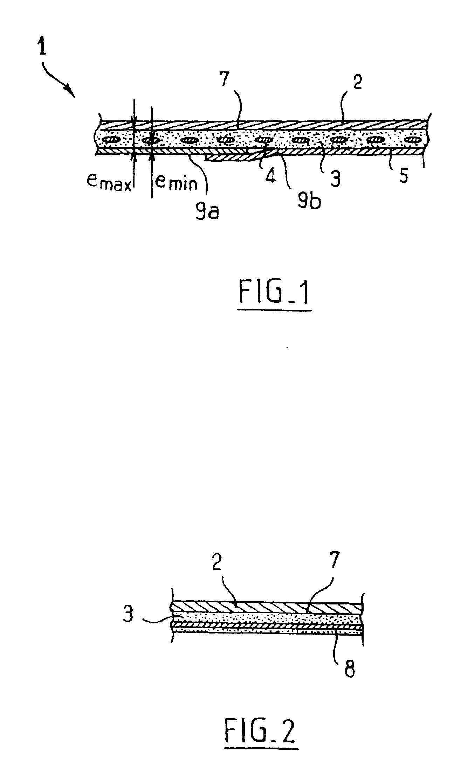

On a représenté sur la figure 1 un patch 1 conforme à un premier exemple de réalisation de l'invention, comportant une feuille de support 2 revêtue sur une face d'une matrice 3 réalisée dans un matériau ayant des propriétés auto-adhésives.

La feuille de support 2 peut avoir subi un traitement corona visant à améliorer l'adhérence de la matrice.

Une structure ajourée constituée ici par un filet 4 en polyamide est noyée dans la matrice 3 à proximité de sa surface 5 destinée à venir au contact de la peau.

La structure ajourée utilisée est avantageusement hydrophobe et sans aptitude particulière à absorber l'humidité et/ou les substances actives éventuellement contenues dans la matrice 3. Ainsi, elle n'est pas susceptible de se gorger d'eau ou de substances actives au cours de l'utilisation du patch, ce qui pourrait diminuer l'adhérence du patch et/ou retarder la diffusion desdites substances dans la peau.

On notera que la présence du filet 4 à proximité de la surface du patch destinée à venir au contact de la peau n'affecte pas l'accrochage de la matrice sur la feuille de support.

Dans l'exemple décrit, la matrice 3 présente une épaisseur totale eₘₐₓ constante, comprise par exemple entre 0,1 et 3 mm.

Le filet 4 se situe à une distance eₘᵢₙ de la surface 5, et ses brins définissent avec celle-ci des zones de moindre épaisseur, donc de moindre adhérence du matériau constituant la matrice 3.

De préférence, le rapport eₘᵢₙ/eₘₐₓ est inférieur ou égal à 1/10.

Il résulte de la présence du filet 4 au sein de la matrice 3 des variations localisées de l'adhérence du patch 1 sur la peau et une adhérence globale du patch 1 moindre que si le filet 4 était absent, pour une épaisseur donnée de la matrice 3.

Il est possible de jouer sur le rapport eₘᵢₙ/eₘₐₓ pour adapter l'adhérence du patch 1 en fonction de la sensibilité de la zone sur laquelle il doit être appliqué et le cas échéant de la ou des substances actives incorporées à l'intérieur de la matrice 3.

La feuille de support 2 est réalisée dans un matériau souple, par exemple du polyester, polyéthylène ou polypropylène ou tout autre matériau approprié, occlusif ou non.

Il peut s'avérer avantageux de métalliser la feuille de support 2, de manière à réfléchir le rayonnement thermique du corps vers la surface de la peau, et permettre à la matrice 3 d'être rapidement portée à la température de l'utilisateur, ce qui peut avoir pour conséquence d'augmenter son adhérence et/ou de favoriser la diffusion et/ou l'action de la ou des substances actives éventuellement contenues à l'intérieur.

L'épaisseur de la feuille de support métallisée est comprise par exemple entre 20 et 120 µm.

En variante, la feuille de support pourrait être constituée par une feuille de métal.

Le patch peut également comporter une couche apte à capter l'énergie thermique d'un rayonnement extérieur.

Il peut en outre s'avérer avantageux de colorer d'une couleur foncée la matrice 3 et/ou le filet 4 et/ou la feuille de support 2, de manière à permettre à l'utilisateur, après enlèvement du patch 1, d'observer par contraste la quantité et/ou la nature des impuretés retirées. L'utilisateur peut alors déterminer si une nouvelle application est nécessaire et également le cas échéant, si la fréquence et/ou la nature du traitement doivent être modifiées. Par exemple, on incorpore à la matrice des pigments violets commercialisés par la société RDF Chimie sous la référence DC violet 2K7014.

Le matériau utilisé pour réaliser la matrice 3 comporte avantageusement un ou plusieurs polymères acryliques, vinyliques, polyuréthanne, EPDM ou élastomère dont l'adhérence (mesurée parallèlement aux surfaces en contact) est de préférence comprise entre 300 et 800 g/cm².

Par exemple, on utilise un adhésif acrylique en base solvant (éthyle acétate hexane éthanol), auto-réticulant, sensible à la pression, dont l'adhérence initiale est de 100g/cm² environ, et le pouvoir adhésif (TAC) après un temps de pose suffisant est de 300g/cm² environ. Un tel adhésif est commercialisé par la société MAPEI sous la dénomination AGXL.

L'utilisation d'une feuille de support métallisée permet de réduire la durée nécessaire à cet adhésif pour atteindre un pouvoir adhésif proche du pouvoir adhésif maximum, par concentration de la chaleur au niveau de l'interface peau/matrice.

Grâce à la présence du filet 4, le pouvoir adhésif du matériau utilisé pour réaliser la matrice 3 peut être réduit de 50 % ou plus, par exemple de 600 g/cm² sans filet à 200 g/cm² voire 150 g/cm² en présence du filet.

La matrice 3 comporte avantageusement une ou plusieurs substances actives ayant un effet sur la peau telles que par exemple des agents anti-oxydants, anti-radicaux libres, hydratants, dépigmentants, liporégulateurs, anti-acnéiques, anti-séborrhéiques, anti-vieillissement, adoucissants, anti-rides, kératolitiques, anti-inflammatoires, rafraîchissants, cicatrisants, protecteurs vasculaires, anti-bactériens, anti-fongiques, anti-perspirants, déodorants, conditionneurs de la peau, insensibilisants, immunomodulateurs et nourrissants, absorbeurs d'humidité (coton, polyacrylate), de sébum (Orgasol®).

De préférence, le patch 1 est revêtu avant utilisation, sur sa surface destinée à venir au contact de la peau, d'une pellicule de protection amovible.

Cette pellicule de protection comporte de préférence deux parties 9a, 9b, se chevauchant dans la région médiane du patch, de manière à permettre l'utilisateur de les retirer sans toucher des doigts la matrice 3, donc sans lui faire perdre de son pouvoir adhésif.

Le patch selon l'invention peut être fabriqué en enduisant le filet 4 avec le matériau destiné à constituer la matrice 3, alors imbibé d'un ou plusieurs solvants, en amenant ensuite l'ensemble au contact d'un support résistant à la température, puis en faisant passer le tout dans un four destiné à évaporer le ou les solvants précités.

A la sortie du four le support est séparé de la matrice 3 devenue solidaire du filet 4 et ces derniers sont appliqués sur la face 7 de la feuille de support 2, l'ensemble étant ensuite calandré.

Il faut bien comprendre que l'invention n'est pas limitée au seul emploi d'un filet 4 tel que décrit pour réaliser la structure ajourée. On peut notamment utiliser toute autre structure perméable, un film perforé, un tissé ou non tissé par exemple.

On a représenté sur la figure 2 un patch conforme à un deuxième exemple de réalisation de l'invention.

Ce patch diffère de celui décrit en référence à la figure 1 par le fait que la structure perméable utilisée n'est pas un filet mais un non-tissé 8 en fibres de polyéthylène, au travers duquel peuvent diffuser le cas échéant une ou plusieurs substances contenues dans la matrice.

Ce non-tissé 8 est positionné au sein de la matrice 3 de manière à modifier le pouvoir adhésif global de celle-ci et obtenir l'adhérence recherchée.

Le pouvoir adhésif global du patch peut encore être modifié, le cas échéant, par la présence de reliefs à la surface de la matrice.

On peut utiliser pour former ces reliefs une pellicule de protection gaufrée qui laisse sur la matrice, après son enlèvement, les reliefs voulus.

Le patch 1 est de préférence offert à l'utilisateur à l'état pré-découpé, de manière à épouser la forme de la région du corps à traiter, sa taille étant comprise par exemple entre 1 et 30 cm².

De préférence, le patch est conditionné dans un sachet de protection formé de deux feuilles d'un complexe papier/film en matière plastique étanche, par exemple du polypropylène, le papier étant revêtu d'un adhésif scellable à froid, les feuilles étant scellées autour du patch par contact des faces enduites d'adhésif.

Un tel conditionnement permet de maintenir le patch à l'abri de l'air et d'améliorer ses conditions de conservation.

La durée d'application du patch sur la peau est par exemple de 30s à 5mn, et de préférence de 1 mn à 5mn.

## Revendications

1. Patch comprenant une matrice (3) réalisée dans un matériau ayant des propriétés auto-adhésives avant l'application sur la peau, **caractérisé par le fait qu'**il comporte une structure perméable (4;8) noyée entièrement dans ladite matrice (3) à proximité de sa surface de manière à modifier son pouvoir adhésif global.

2. Patch selon la revendication 1, **caractérisé par le fait que** ladite structure perméable produit des variations locales du pouvoir adhésif de la matrice (3) et de préférence est constituée par un filet (4).

3. Patch selon la revendication 1, **caractérisé par le fait que** la structure perméable est constituée par un non-tissé (8).

4. Patch selon l'une quelconque des revendications 1 à 3, **caractérisé par le fait qu'**il comprend dans ladite matrice (3) au moins une substance active ayant un effet sur la peau.

5. Patch selon l'une quelconque des revendications précédentes, **caractérisé par le fait que** ladite matrice (3) comporte un ou plusieurs polymères acryliques ou vinyliques.

6. Patch selon l'une quelconque des revendications précédentes, **caractérisé par le fait que** ladite structure perméable (4;8) présente une couleur foncée.

7. Patch selon l'une quelconque des revendications précédentes, **caractérisé par le fait que** ladite matrice (3) présente une couleur foncée.

8. Patch selon l'une quelconque des revendications précédentes, **caractérisé par le fait que** ladite matrice (3) est portée par une feuille de support (2) de couleur foncée au moins sur sa face (7) tournée vers ladite matrice (3).

9. Patch selon l'une quelconque des revendications 1 à 8, **caractérisé par le fait que** ladite matrice (3) est portée par une feuille de métal ou une feuille de support (2) métallisée.

10. Patch selon l'une quelconque des revendications précédentes, **caractérisé par le fait que** sa taille est comprise entre 1 et 30 cm² et **par le fait qu'**il est pré-découpé de manière à épouser la forme de la région du corps à traiter.

11. Patch selon l'une quelconque des revendications précédentes, **caractérisé par le fait que** le rapport de l'épaisseur (eₘᵢₙ) du matériau constituant ladite matrice (3) entre la structure perméable (4) et la surface (5) destinée à être appliquée sur la peau à l'épaisseur (eₘₐₓ) totale de ladite matrice (3) est inférieur ou égal à 1/10.

12. Patch selon l'une quelconque des revendications précédentes, **caractérisé par le fait que** le matériau utilisé pour constituer la matrice (3) présente une adhérence comprise entre 300 et 600 g/cm².

13. Patch selon l'une quelconque des revendications précédentes, **caractérisé par le fait que** la matrice est revêtue avant l'utilisation d'une pellicule de protection comprenant deux parties (9a, 9b) se chevauchant dans la région médiane du patch.

14. Patch selon l'une quelconque des revendications précédentes, **caractérisé par le fait qu'**il comporte une couche apte à capter l'énergie thermique du rayonnement extérieur.

15. Patch selon l'une quelconque des revendications précédentes, **caractérisé par le fait qu'**il est conditionné dans un sachet de protection formé de deux feuilles d'un complexe papier/film en matière plastique étanche, le papier étant revêtu d'un adhésif scellable à froid, les feuilles étant scellées autour du patch par contact des faces enduites d'adhésif.

16. Patch selon la revendication 2, **caractérisé par le fait que** le filet (4) n'affecte pas l'accrochage de la matrice (3) sur une feuille de support.

17. Utilisation non-médicale du patch tel que défini dans l'une quelconque des revendications 1 à 16, **caractérisé par le fait qu'**il est laissé sur la peau pendant une durée d'application comprise entre 30 s et 5 mn.

## Claims

1. A patch comprising a matrix (3) made of a material having self-adhesive properties prior to application to the skin, the patch being **characterized by** the fact that it includes a permeable structure (4; 8) completely embedded in said matrix (3) close to its surface so as to modify its overall adhesive power.

2. A patch according to claim 1, **characterized by** the fact that said permeable structure produces local variations in the adhesive power of the matrix (3) and is preferably constituted by a net (4).

3. A patch according to claim 1, **characterized by** the fact that the permeable structure is constituted by a non-woven cloth (8).

4. A patch according to any one of claims 1 to 3, **characterized by** the fact that in said matrix (3) it includes at least one active substance having an effect on the skin.

5. A patch according to any preceding claim, **characterized by** the fact that said matrix (3) comprises one or more acrylic or vinyl polymers.

6. A patch according to any preceding claim, **characterized by** the fact that said permeable structure (4; 8) is of a dark color.

7. A patch according to any preceding claim, **characterized by** the fact that said matrix (3) has a dark color.

8. A patch according to any preceding claim, **characterized by** the fact that said matrix (3) is carried by a backing sheet (2) of dark color, at least on its face (7) facing towards said matrix (3).

9. A patch according to any one of claims 1 to 8, **characterized by** the fact that said matrix (3) is carried by a metal foil or a metallized backing sheet (2).

10. A patch according to any preceding claim, **characterized by** the fact that its size lies in the range 1 cm² to 30 cm², and by the fact that it is pre-cutout so as to match the shape of the region of the body to be treated.

11. A patch according to any preceding claim, **characterized by** the fact that the ratio of the thickness (eₘᵢₙ) of the material constituting said matrix (3) between the permeable structure (4) and the surface (5) that is to be pressed against the skin over the total thickness (eₘₐₓ) of said matrix (3) is less than or equal to 1/10.

12. A patch according to any preceding claim, **characterized by** the fact that the material used for making the matrix (3) has adhesion lying in the range 300 g/cm² to 600 g/cm².

13. A patch according to any preceding claim, **characterized by** the fact that the matrix is coated prior to use with a protective membrane comprising two portions (9a, 9b) that overlap in the middle region of the patch.

14. A patch according to any preceding claim, **characterized by** the fact that it includes a layer suitable for picking up heat energy from external radiation.

15. A patch according to any preceding claim, **characterized by** the fact that it is packaged in a protective sachet made up of two sheets of a leakproof laminate of paper and plastics material film, the paper being coated in an adhesive that operates cold, the sheets being sealed around the patch by putting their faces that are coated in adhesive into contact.

16. A patch according to claim 2, **characterized by** the fact that the net (4) has no effect on the adhesion of matrix (3) on a backing sheet.

17. The non medical use of a patch as defined in any one of claims 1 to 16, **characterized by** the fact that it is left on the skin for a period of application that lies in the range 30 seconds to 5 minutes.

## Patentansprüche

1. Pflaster, umfassend eine Matrix (3), realisiert aus einem Material mit selbsthaftenden Eigenschaften vor der Anwendung auf der Haut, **dadurch gekennzeichnet, dass** es eine permeable Struktur (4; 8) trägt, vollständig in die Matrix (3) in der Nähe ihrer Oberfläche eingetaucht, um deren Gesamthaftungsvermögen zu modifizieren.

2. Pflaster gemäß Anspruch 1, **dadurch gekennzeichnet, dass** die permeable Struktur lokale Variationen des Haftungsvermögens der Matrix (3) erzeugt und vorzugsweise aus einem Faden bzw. Netz (4) besteht.

3. Pflaster gemäß Anspruch 1, **dadurch gekennzeichnet, dass** die permeable Struktur aus einem nicht-gewebten Gewebe (8) besteht.

4. Pflaster gemäß einem der Ansprüche 1 bis 3, **dadurch gekennzeichnet, dass** es in der Matrix (3) mindestens eine aktive Substanz mit einer Wirkung auf die Haut enthält.

5. Pflaster gemäß einem der vorstehenden Ansprüche, **dadurch gekennzeichnet, dass** die Matrix (3) eines oder mehrere Acrylsäure- oder Vinylpolymere enthält.

6. Pflaster gemäß einem der vorstehenden Ansprüche, **dadurch gekennzeichnet, dass** die permeable Struktur (4; 8) eine dunkle Farbe aufweist.

7. Pflaster gemäß einem der vorstehenden Ansprüche, **dadurch gekennzeichnet, dass** die Matrix (3) eine dunkle Farbe aufweist.

8. Pflaster gemäß einem der vorstehenden Ansprüche, **dadurch gekennzeichnet, dass** die Matrix (3) von einer Trägerfolie (2) mit einer dunklen Farbe auf mindestens derjenigen Oberfläche (7), die der Matrix (3) zugewandt ist, geträgert wird.

9. Pflaster gemäß einem der Ansprüche 1 bis 8, **dadurch gekennzeichnet, dass** die Matrix (3) auf einer Metallfolie oder einer metallisierten Trägerfolie (2) geträgert ist.

10. Pflaster gemäß einem der vorstehenden Ansprüche, **dadurch gekennzeichnet, dass** dessen Größe zwischen 1 und 30 cm² liegt, und dadurch, dass es so vorgeschnitten ist, dass es die Form des zu behandelnden Körperbereiches annimmt.

11. Pflaster gemäß einem der vorstehenden Ansprüche, **dadurch gekennzeichnet, dass** das Verhältnis der Dicke (eₘᵢₙ) des die Matrix (3) aufbauenden Materials zwischen der permeablen Struktur (4) und der Oberfläche (5), bestimmt zum Auftrag auf die Haut, und der Gesamtdicke (eₘₐₓ) der Matrix (3) kleiner oder gleich 1/10 ist.

12. Pflaster gemäß einem der vorstehenden Ansprüche, **dadurch gekennzeichnet, dass** das zum Aufbau der Matrix (3) verwendete Material eine Haftung zwischen 300 und 600 g/cm² aufweist.

13. Pflaster gemäß einem der vorstehenden Ansprüche, **dadurch gekennzeichnet, dass** die Matrix vor der Anwendung durch eine Schutzabdeckung bedeckt ist, umfassend zwei Teile (9a, 9b), die im mittleren Bereich des Pflasters übereinander greifen.

14. Pflaster gemäß einem der vorstehenden Ansprüche, **dadurch gekennzeichnet, dass** eine Schicht trägt, bestimmt zum Aufnehmen von thermischer Energie äußerer Strahlung.

15. Pflaster gemäß einem der vorstehenden Ansprüche, **dadurch gekennzeichnet, dass** es in einer Schutzhülle konditioniert ist, die aus zwei Bögen eines Komplexes aus Papier/wasserdichtem Plastikfilm gebildet ist, wobei das Papier mit einem im Kalten versiegelbaren Adhesiv bedeckt ist, wobei die Bögen um das Pflaster herum durch Kontakt der mit Adhesiv bedeckten Flächen versiegelt sind.

16. Pflaster gemäß Anspruch 2, **dadurch gekennzeichnet, dass** die Faser oder das Netz (4) die Befestigung der Matrix (3) auf einer Trägerfolie nicht beeinträchtigt.

17. Nicht-medizinische Verwendung des Pflasters, wie in einem der Ansprüche 1 bis 16 definiert, **dadurch gekennzeichnet, dass** man es für eine Anwendungsdauer zwischen 30 Sekunden und 5 Minuten auf der Haut belässt.
